(19) **Europäisches Patentamt European Patent Office Office européen des brevets**

(11) **EP 3 791 198 B1**

(12) **EUROPEAN PATENT SPECIFICATION**

(45) Date of publication and mention of the grant of the patent:
**07.02.2024 Bulletin 2024/06**

(21) Application number: **18917842.9**

(22) Date of filing: **11.05.2018**

(51) International Patent Classification (IPC):
*G01R 33/00* (2006.01)   *G01R 33/12* (2006.01)
*A61B 5/05* (2021.01)   *A61B 5/0515* (2021.01)

(52) Cooperative Patent Classification (CPC):
**A61B 5/0515; G01R 33/0047; G01R 33/12;**
A61B 2560/0223

(86) International application number:
**PCT/TR2018/050225**

(87) International publication number:
**WO 2019/216839 (14.11.2019 Gazette 2019/46)**

(54) **METHOD OF CALIBRATING MAGNETIC PARTICLE IMAGING SYSTEM**

VERFAHREN ZUR KALIBRIERUNG EINES MAGNETTEILCHENBILDGEBUNGSSYSTEMS

PROCÉDÉ D'ÉTALONNAGE DE SYSTÈME D'IMAGERIE À PARTICULES MAGNÉTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**17.03.2021 Bulletin 2021/11**

(73) Proprietors:
• **Aselsan Elektronik Sanayi ve Ticaret Anonim Sirketi**
**06370 Ankara (TR)**
• **Ihsan Dogramaci Bilkent Üniversitesi**
**06800 Cankaya/Ankara (TR)**

(72) Inventors:
• **TOP, Can Baris**
**Yenimahalle/Ankara (TR)**
• **ILBEY, Serhat**
**Yenimahalle/Ankara (TR)**
• **GÜNGÖR, Alper**
**Yenimahalle/Ankara (TR)**
• **GÜVEN, Hüseyin Emre**
**Yenimahalle/Ankara (TR)**
• **ÇUKUR, Tolga**
**06800 Çankaya/Ankara (TR)**
• **SARITAS ÇUKUR, Emìne Ulku**
**06800 Çankaya/Ankara (TR)**

(74) Representative: **Yamankaradeniz, Kemal**
**Destek Patent, Inc.**
**Maslak Mah.,**
**Büyükdere Cad., No: 243,**
**Kat: 13, Spine Tower, Sariyer,**
**34485 Istanbul (TR)**

(56) References cited:
**US-A1- 2015 221 103     US-A1- 2017 020 407**

• **ILBEY SERHAT ET AL: "Coded scenes for fast system calibration in magnetic particle imaging", 2018 IEEE 15TH INTERNATIONAL SYMPOSIUM ON BIOMEDICAL IMAGING (ISBI 2018), IEEE, 4 April 2018 (2018-04-04), pages 315-318, XP033348160, DOI: 10.1109/ISBI.2018.8363582 [retrieved on 2018-05-23]**
• **SERHAT ILBEY: "Image reconstruction for Magnetic Particle Imaging using an Augmented Lagrangian Method", IEEE 14th International Symposium on Biomedical Imaging, 18 April 2017 (2017-04-18), pages 64-67, XP055651841,**
• **SERHAT ILBEY: "Comparison of System-Matrix-Based and Projection-Based Reconstructions for Field Free Line Magnetic Particle Imaging", International Journal on Magnetic Particle Imaging, vol. 3, no. 1, 29 March 2017 (2017-03-29), XP055651843,**

- **H. EMRE GUVEN: "An Augmented Lagrangian Method for Complex-Valued Compressed SAR Imaging", IEEE Transactions on Computational Imaging, vol. 2, no. 3, 13 June 2016 (2016-06-13), XP011618760,**

## Description

### Technical Field

The present disclosure relates a method of calibrating magnetic particle imaging system by proposing a coded calibration scene that contains multiple nanoparticle samples distributed inside its volume, larger than the field of view on which the scene is moved linearly in one or more directions and/or rotated at one or more axes, and further, a mechanical system for moving the coded calibration scene.

### Related Art

Magnetic nanoparticles can be used for various purposes in medicine such as angiography, stem cell tracking, imaging of cancerous cells and targeted drugs. Magnetic nanoparticles can be imaged non-invasively using the Magnetic Particle Imaging (MPI) method. Two different methods are used as standard for image reconstruction in the magnetic particle imaging method.

The first one is the system calibration method as stated in US8355771B2 numbered patent, in which a small volume nanoparticle sample is scanned mechanically at the desired system resolution steps in the field of view to obtain the calibration data of the system [1]. Images are generated using this calibration data (which is also called the system matrix). In the standard system calibration method, the calibration measurements last very long since the sample nanoparticle must be mechanically scanned and measured at every grid point in the field of view. The mechanical scanning time from one point to the other and acquisition of the measurement data takes about 1.3 sec [2]. For a small field of view having 30 x 30 x 30 grid points, the calibration time lasts 9.75 hours. In clinical practice, the calibration of a larger imaging volume may last for months. There is a need to calibrate the system frequently, since nanoparticle characteristics are known to vary from batch to batch and is also affected by the imaging sequence. For this reason, standard system calibration method cannot be practically adopted for systems with large field of view. In addition, since the nanoparticle to be scanned must be smaller than or equal to the voxel size of the image, the number of nanoparticles in the scanned sample is limited and the signal-to-noise ratio is small. A method to increase the signal-to-noise ratio is multiple data acquisition at the same position, and averaging. Therefore, the mechanical motion cannot be continuous, and the scanner should be stopped at every grid point, and moved to the next point after taking enough measurements to reach to the desired signal noise level. This limits the speed of the calibration measurements.

Recently, a calibration method has been proposed in an application no. US20150221103A1, in which the nanoparticle sample is scanned at random positions much fewer than the total number of voxels in the field of view. This is possible according to the compressed sensing theory [3] since the system matrix is sparse in certain transform domains (discrete Fourier, cosine, or Chebyshev). It has been shown that this method can reduce the number of scanned points by 80-90%. Instead of taking measurements from all of the voxels (N) in the field of view, system calibration can also be done by making reduced number of measurements at random M (<N) voxel positions using compressed sensing techniques. Since it is not possible to calculate how small M should be analytically, the M / N ratio should be chosen according to the image quality. Experimental images were obtained in the above mentioned reference. While image quality was acceptable for M / N = 0.1, it was significantly degraded for lower M / N ratios. The calibration time can be reduced by a factor of 10 with this method but very long calibration times are still needed since the sample is mechanical scanned, i.e. a measurement area of 200 x 200 x 200 points will take longer than 10 days to measure.

The second reconstruction method is the X-space approach used in application numbered EP3143929A1. In this method, there is no calibration step; images are generated using the signal equation model for the magnetic particles imaging. Image reconstruction is done in the time domain by using the MPI signal equation. In this method, deviations from the ideal of MPI hardware are not taken into account and the resolution is lower than the system calibration method.

The application numbered US20170020407A1 presents a magnetic particle imaging (MPI) method that determines calibration and measurement volumes, wherein the calibration volume is larger than the measurement volume and the overall measurement volume is arranged within the calibration volume. The proposed calibration region is a few voxels larger region than the measurement region. The underlining reason is that the magnetic particle response does not die out sharply at the edge of the measurement region. By measuring the magnetic particle sample outside the measurement region, unwanted artifacts caused by the particles outside the measurement region are reduced.

S. Ilbey et al. [9] suggests a new method based on Alternating Direction Method of Multipliers (a subset of Augmented Lagrangian Methods, ADMM) with total variation and l1 norm minimization, to reconstruct MPI images. The method is demonstrated on data simulated for a field free line MPI system and its performance is compared against the conventional Algebraic Reconstruction Technique. The ADMM improves image quality as indicated by a higher structural similarity, for low signal-to-noise ratio datasets, and it significantly reduces computation time.

S. Ilbey et al. [10] proposes the use of coded scenes that include MNP samples at multiple positions inside the FOV, and reconstruct the SFM using compressed sensing techniques.

A von Gladiss et al. [2] discloses an electronic calibration method for accelerating the calibration procedure. The nanoparticle sample is placed in a separate calibration unit, which can generate homogeneous magnetic fields of any orientation imitating the magnetic fields that the nanoparticle sample would be exposed in the MPI system. Although this method provides faster calibration than the standard method, it requires the use of a separate calibration unit; the magnetic field distribution of the MPI system must be separately measured in the field of view; and the calibration unit measurements must be related to the MPI system measurements. Since magnetic field distribution measurements of the MPI system require mechanical scanning at each voxel in the field of view, as in the case of standard system calibration measurements, the advantage of electronic calibration is limited.

## Purpose of the Invention

In the present invention, a large calibration apparatus, which will be referred as a coded calibration scene, is proposed for the calibration of an MPI system. Coded calibration scene includes nanoparticle samples at multiple positions. It is moved linearly in one or more directions, and/or rotated about one or more axes. During this movement, calibration measurement data are acquired at certain positions of the coded calibration scene. System matrix is generated using this measurement data with compressed sensing methods. The advantages that distinguish this method from other available methods are listed below:

In the state of the art, US patent no. US20150221103A1, a single nanoparticle sample is mechanically scanned for M voxels selected randomly or pseudo-randomly from the total number of voxels (N) in the field of view one by one for MPI system calibration. In the present invention, a calibration apparatus is proposed, which includes multiple nanoparticle samples and is larger than the field of view of the imaging system at least in one direction. Thus, the level of the received signal is much higher compared to the level of the signal received from a single nanoparticle sample. This allows the measurements to be made during continuous movement of the calibration scene, speeding up the calibration procedure substantially. In addition, the information content of the each measurement is increased as the nanoparticle samples at different positions are measured at the same time in a single measurement. Therefore, the system calibration matrix can be formed using fewer measurements. This provides an advantage for systems with large field of view.

In the method proposed by A von Gladiss *et al.* [2], a separate calibration unit is used for nanoparticle characterization. Therefore, the measurement of the magnetic field in the field of view of the MPI system is necessary. In the present invention, all the effects (magnetic field inhomogeneities, nanoparticle response) are taken into account in a single calibration scan.

## Brief Description of the Drawings

Figure 1 shows the cross section of the bore of a magnetic particle imaging setup, the nonhomogeneous primary magnetic field with two zones and homogeneous secondary magnetic field, and the field of view.

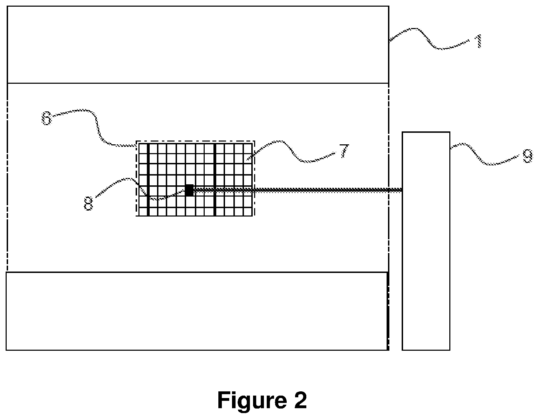

Figure 2 shows an entire field of view that is hypothetically divided into small voxels and a calibration setup using a sample containing the nanoparticles.

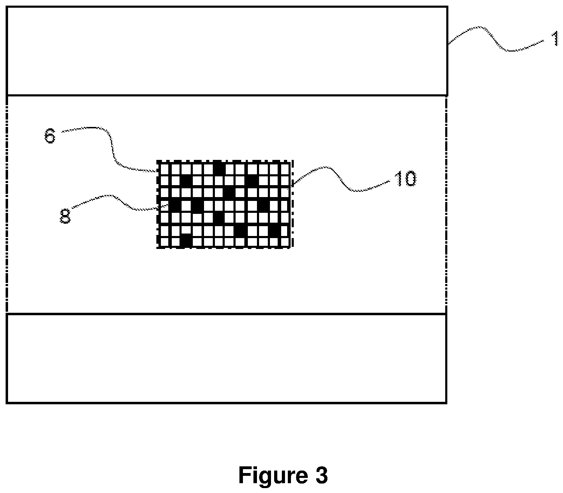

Figure 3 shows a coded calibration scene with a plurality of nanoparticle samples distributed randomly or pseudo-randomly inside its volume.

Figure 4 shows comparison of the standard compressed sensing method with the proposed method for the same noise level using a simulation model. Proposed method shows better image quality with smaller number of measurements (M).

Figure 5 and 6 show nanoparticle locations of a spherical calibration scene at 0 and 45 degree angles, respectively.

Figure 7 shows a spherical calibration scene that rotates in one axis and slides in another axis.

Figure 8 shows a calibration stage and a rotating mechanism rotate about different rotational axes.

Figure 9 and 10 show a spherical calibration scene and an external mechanism for linear and rotational movement of the spherical calibration scene in top and side views, respectively.

Figure 11 shows a calibration scene including nanoparticle chambers connected to each other by thin channels and one or more points for being filled in or discharged.

Figure 12 shows a spherical scene with rod shaped nanoparticle specimens.

Figure 13 shows a calibration scene designed as a long rectangular prism making linear motion on a sliding belt.

Figures 14 and 15 show a cylindrical calibration scene and an external mechanism for linear and rotational movement of the scene from the top and the side, respectively.

Figure 16 shows a cylindrical calibration scene with columnar cavities for nanoparticle samples.

Figure 17 shows a cylindrical calibration scene with a thin tube in the form of a complex curve in three dimensions with an input and an output.

**Part References**

1. MPI system

2. Primary magnetic field

3. First zone of the primary magnetic field

4. Second zone of the primary magnetic field

5. Secondary magnetic field

6. Field of view

7. Voxel

8. Magnetic nanoparticle sample

9. Mechanical scanner

10. Coded calibration scene

11. Spherical calibration scene

12. Rotation center

13. Mechanism for translating and rotating a calibration scene around one axis

14. Mechanism for translating and rotating a calibration scene around two axes

15. Auxiliary mechanical system for translating and rotating a calibration scene

16. Railed slide

17. Rotation axis

18. Thin channel

19. Opening

20. Rod shaped nanoparticle sample

21. Rectangular prism calibration scene

22. Sliding belt

23. Optical reflector

24. Laser tracker

25. Cylindrical calibration scene

26. Columnar cavity

27. Input for filling the magnetic nanoparticles inside the calibration scene

28. Output for draining the magnetic nanoparticles inside the calibration scene

29. Thin tube

## Detailed Description

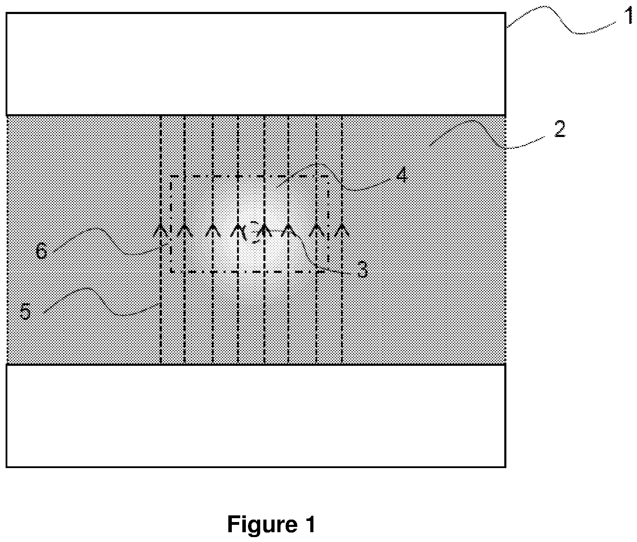

In an MPI system (1) that consists of a magnetic field generator and a measurement device as shown in Figure 1, the distribution of magnetic nanoparticles is imaged using a nonhomogeneous primary magnetic field (2) having two zones [4]. The first (3) of these two zones has a very low magnetic field intensity and is called the field free region (FFR). The magnetic nanoparticles in the FFR can be magnetized in the direction of a secondary external magnetic field (5). In the second zone (4), the magnetic field intensity is high and the magnetic nanoparticles in this region are in saturation. Therefore, they respond marginally to a secondary magnetic field (5). The secondary magnetic field (5) is applied to the entire field of view (6) as a time varying magnetic field. The time-dependent magnetization of the magnetic nanoparticles in the FFR is measured by the receiving coil(s). The amplitude of the measured signal is directly proportional to the number of nanoparticles in the FFR. The FFR is scanned electronically or mechanically throughout the field of view (6) to obtain the nanoparticle distribution in the field of view (6). Since the magnetic nanoparticles have a non-linear magnetization curve, the received signal from the particles in the FFR contains the harmonics of the frequency of the transmitted signal. The received signal properties depend on the properties of the nanoparticle (size, shape, material, etc.) and the nanoparticle environment (viscosity, temperature), and properties of the magnetic field of the imaging system. In MPI, best image quality is achieved with the image reconstruction method based on the system calibration method, which takes all these effects into account [5].

In the system calibration image reconstruction method, firstly the entire field of view (6) is hypothetically divided into small voxels (7). A system matrix is formed using a sample (8) filled with a magnetic nanoparticle having a size of a voxel (7). For this, the sample (8) containing the nanoparticles is scanned to every voxel position by means of a mechanical scanner (9). Secondary magnetic field signal is applied, and the nanoparticle signal received by the receiving coils is stored in a digital storage unit (e.g. hard disk). In practice, the measurement data are acquired multiple times at the same voxel point, and the signal to noise ratio is increased by averaging the measurements data. The measured signal from a single voxel is converted to the frequency domain using the Fourier transform, forming a column of the system matrix ($A$). The whole system matrix is generated by taking measurements at all voxel positions. This process is called the calibration step.

For imaging, measurement data are acquired by scanning the FFR inside the object, and the image is reconstructed using this measurement data and the system matrix. To this end, a linear equation set $Ax = b$ is solved. In this equation set, $A$ is the system matrix, b is the vector of measurements taken from the object, and x is the nanoparticle distribution inside the object. The major disadvantage of the system matrix calibration method is its long duration (about 1.3 seconds per voxel, multiplied by the number of voxels) [2]. In addition, since the sample size of the nanoparticle is very small, the signal level is low and it is necessary to increase the signal-to-noise ratio by taking multiple measurements. This prevents continuous mechanical scanning, leading to the prolongation of the measurement period.

The present invention proposes the use of coded calibration scenes (10) to solve the problems of the prior art. A coded calibration scene can be defined as an apparatus containing a plural number of nanoparticle samples, distributed inside its volume. This method has the following advantages: The signal level increases proportional to the number of particles used in the calibration scan, and the condition of the compressed sensing problem is increased [6]. As a result, calibration is possible with fewer number of measurements using compressed sensing algorithms such as greedy reconstruction algorithms, approximate message passing, optimization based techniques, etc. [3].

According to the compressed sensing theory, the correlation of calibration scenes with each other should be minimized. For this reason, nanoparticles can be distributed randomly or pseudo-randomly in each calibration scene.

An implementation of this method is as follows: the number of calibration scenes, M, to be measured is predetermined. For this, the simulation model of the imaging system can be used, or a number of calibration scenes are produced during the system tests of the produced imaging system; new scenes are measured until the image quality reaches a sufficient level from the clinical point of view. The measurement data are collected and recorded for M coded calibration scenes. Once these measurements have been taken, the system matrix, $A$, is reconstructed using the following optimization problem:

$$\operatorname*{argmin}_{A} \|DA^T\|_1 , subject\ to\ \|PA^T - A_p\|_2 < \varepsilon_p$$

,subject to $\|PA^T - A_p\|_2 < \varepsilon_p$ where $P$ is the nanoparticle density matrix for the measured coded calibration scenes, $D$ is the transformation matrix associated with a sparsifying transform such as discrete Fourier transform, discrete Chebyshev transform, discrete cosine transform, or any other transform where the vector can be represented with fewer coefficients than its original domain; $A_p$ is the measurement matrix converted to Fourier space for each measurement position; $\varepsilon_p$ represents a constant related to the error caused by the system noise. Different algorithms in the literature can be used to solve the above optimization problem (e.g. Fast Iterative Shrinkage Thresholding Algorithm (FISTA), Alternating Direction Method of Multipliers (ADMM) [7]). Moreover, adding similar regularization functions or using the unconstrained form will not change the described benefits of the invention.

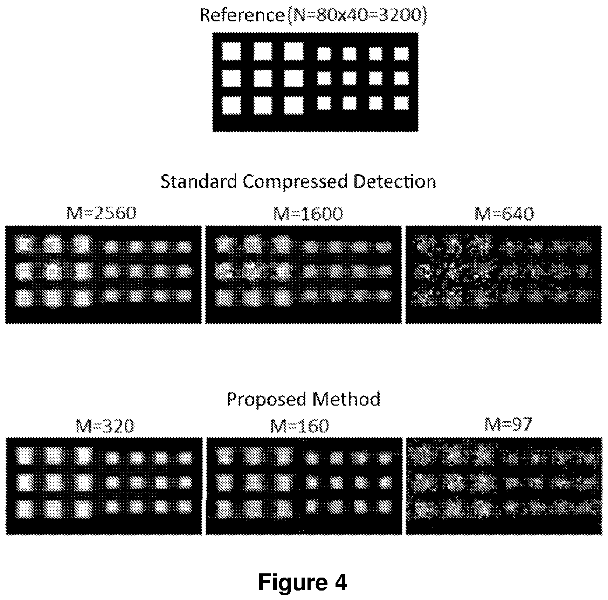

This method is compared with the standard compressed sensing method for the same noise level using a simulation model as revealed in Figure 4. An object with N = 3200 pixels was imaged both by using the standard compressed sensing calibration method with M = 2560 calibration points and M = 320 coded calibration scenes. The resultant image quality was poor for the standard compressed sensing method, while a high quality image was obtained with the coded calibration scenes.

In an embodiment, random points expressed by *P* can be selected from a domain that can be quickly transformed, such as the Hadamard matrix, in order to shorten the solution time of the problem given in the inequality. In this case, the *P* matrix can be expressed as a masked unitary transformation. It has previously been shown that the optimization problem can be solved efficiently in situations involving a masked unitary transformed space [8]. By this way, the problem of solution time can be further decreased.

In practice, the time for switching between the coded calibration scenes would be much longer than measurement time of a single coded calibration scene. Therefore, the total calibration duration would be determined by the total number of coded calibration scenes used and the time required for changing (replacement) of the coded calibration scenes. In order to mitigate this problem, a calibration scene that is larger than the field of view at least in one direction is proposed with the present invention. Instead of changing the calibration scenes one by one, the scene is moved linearly in one or more directions and / or rotated at one or more center points. Calibration measurements are taken at certain positions during continuous movement. The nanoparticle distribution in the imaging field of view changes as a function of time. Therefore, at different time instants, a different part of the calibration scene is present in the field of view. In a preferred embodiment, the measurement is taken during continuous motion of the calibration scene. This is possible when the signal noise ratio is high as a result of large number of nanoparticles used in the calibration scene. Consequently, there is no need to repeat and average the measurements. In this way, it is possible to shorten the measurement time substantially. As a result, it is possible to calibrate the system frequently to obtain a high image quality.

The locations of the nanoparticle samples in the calibration scene must be known precisely. The calibration scene can be produced with high-precision production methods and/or can be measured after production with high resolution imaging methods such as X-ray imaging.

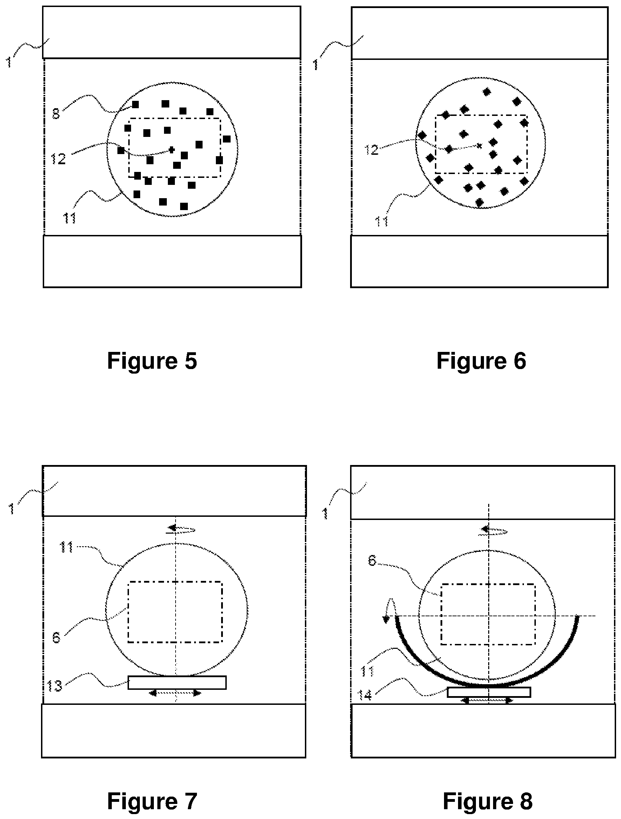

The calibration scene can be moved linearly and/or circularly. In an example embodiment, a spherical calibration scene is rotated about one axis and measurements are taken at K degrees intervals. The position of the nanoparticles samples (8) in the calibration scene change as a function of rotation angle. For example, the nanoparticle locations of a spherical calibration scene (11) at 0 and 45 degree angles are given in Figures 5 and 6, respectively. For each rotation angle, the nanoparticle's new position in the field of view grid, and the nanoparticle density at each grid point in the new location are calculated. The error in this calculation depends on the accuracy of the rotation measurement of the rotation mechanism. If this accuracy is not sufficient, the new position can be precisely measured using a position tracker with high sensitivity, such as a laser tracker or a device of similar purpose. In order to obtain the system matrix with high accuracy, the process can be repeated at a number (L) of different rotation centers (12) to increase the amount of measurement data. The total number of measurements is then M = (360 / K) * L. Once these measurements have been taken, the system matrix is reconstructed by solving the optimization problem given in above stated inequality. In the inequality, P is the matrix containing the nanoparticle density distribution in the field of view at each measurement position.

A mechanism for translating and rotating a calibration scene around one axis (13) can be used to move and/or rotate the calibration scene. The mechanism (13) required to rotate the calibration scene can be designed as an integrated unit or as an external unit to the MPI system (1). An example embodiment is shown in Figure 7. Here, the spherical calibration scene (11) rotates in one axis and slides in another axis. In this way, it is possible to measure the calibration scene at different rotation centers with respect to the field of view center and increase the diversity of the calibration scene measurements. The linear sliding motion as well as the rotation motion can be continuous during the calibration reducing the calibration time compared to stepped motion.

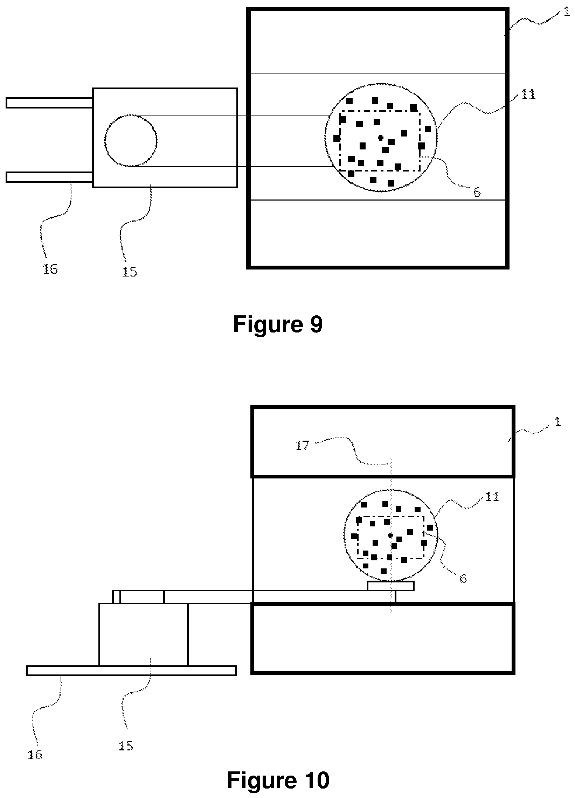

A mechanism for translating and rotating a calibration scene around two axes (14) can also be designed to rotate about different rotational axes as shown in Figure 8. In this case, the conditioning of the autocorrelation of the P matrix can be improved, which is helpful for the solution of the optimization problem. In the implementation of the method, the calibration scene and the rotating mechanism can also be designed as an external unit according to the MPI system's mechanical requirements. Such an implementation is shown in Figure 9 and Figure 10. In Figure 9, a spherical calibration scene (11) is shown in top view, which makes a linear sliding movement on a railed slide (16) and a rotational movement about a rotation axis (17) by means of a reel system. Figure 10 shows the side view of this calibration system. An auxiliary mechanical system for translating and rotating a calibration scene (15) includes the necessary equipment (motor, encoder, motion transfer elements, and computerized control interface) to perform linear and rotational movements of the calibration scene. In a preferred embodiment the mechanical system includes a control unit, which communicates with the MPI system (1) to perform the calibration procedure using the calibration scenes. To this end, the control unit receives the required position of the calibration scene from the MPI system by electronical means, moves the calibration scene to the required position, outputs the position information of the calibration scene obtained from the encoders in the mechanical system and /or tracking device that measure the position of the calibration scene.

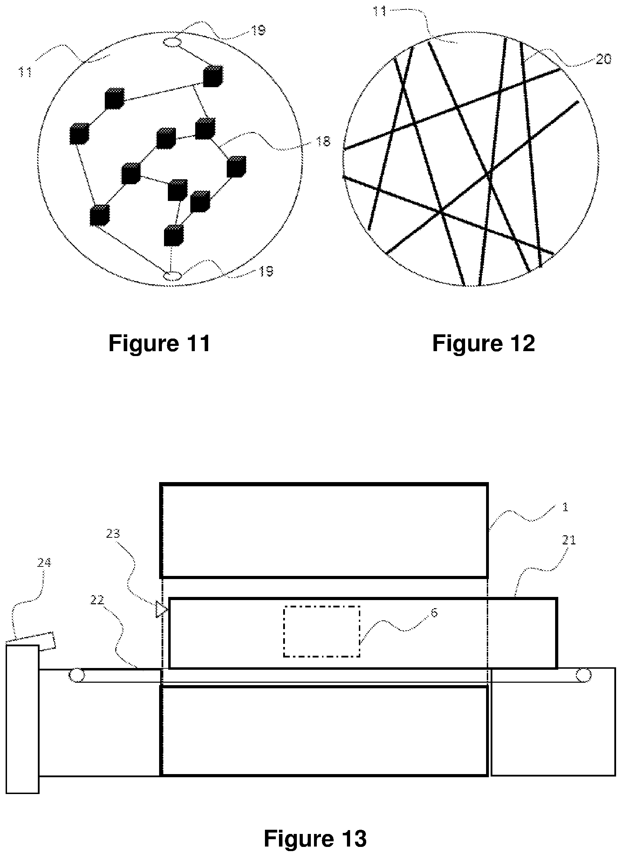

Calibration scenes should allow for rapid filling (and emptying) of different nanoparticles. In an embodiment, a three dimensional coded calibration scene can be formed by a plurality of mechanically separable layers allowing the nanoparticle samples to be changed. In another embodiment, a single layer calibration scene can be used for calibration in two-dimensions. It can be mechanically scanned in the third dimension to calibrate a three dimensional field of view. Figure 11 shows another embodiment; a calibration scene including nanoparticle chambers connected to each other by thin channels (18), and openings (19) for filling or draining the magnetic nanoparticles inside the calibration scene. The calibration scene is a hollow structure with one or more openings for filling or emptying the structure with nanoparticles.

The nanoparticle samples present in the calibration scenes do not have to fit into a single voxel (10). Scenes can include nanoparticle samples of different sizes and shapes. For example, a nanoparticle sample can be of any shape such as spherical, elliptical or rectangular prism, and cover many voxels. In an embodiment, a spherical scene is considered, with rod shaped nanoparticle samples (20) as shown in Figure 12. The rods can be easily taken out and inserted into the scene. The calibration scene can be produced in any arbitrary shape such as sphere, cylinder, cube, rectangular prism.

In another embodiment shown in Figure 13, the calibration scene (21) designed as a long rectangular prism makes only linear motion on a sliding belt (22). Calibration measurements are made at certain positions through the field of view. Figure 13 also shows an optical reflector (23) and a laser tracker (24) to ensure precise measurement of position during movement. One or more reflectors can be attached to the calibration scene for tracking its movement.

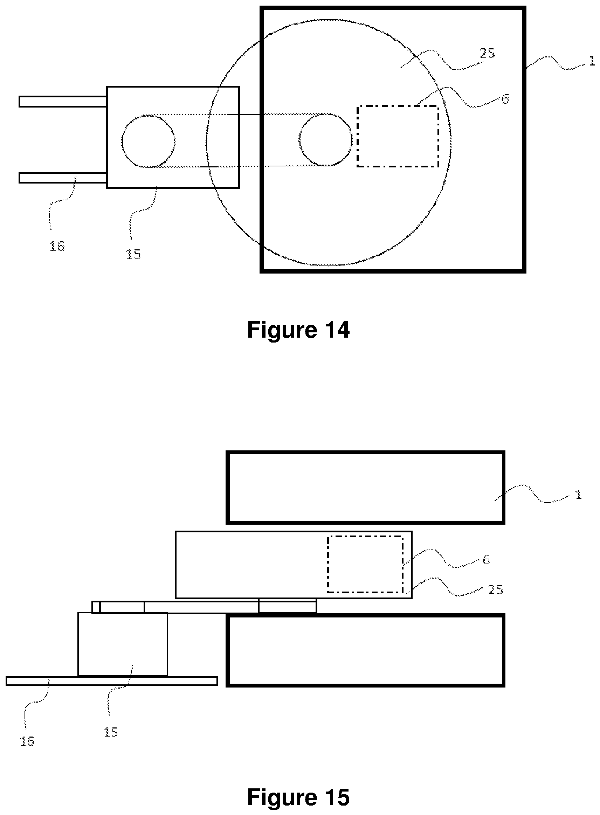

In the embodiments shown in Figures 14 and 15, a cylindrical calibration scene (25) is employed. Calibration can be performed at fewer number of rotations than that required by the calibration scene given in Figures 9 and 10, since the volume of the calibration scene is wider. However, such a calibration scene requires a large opening, which may be suitable for open bore MPI systems.

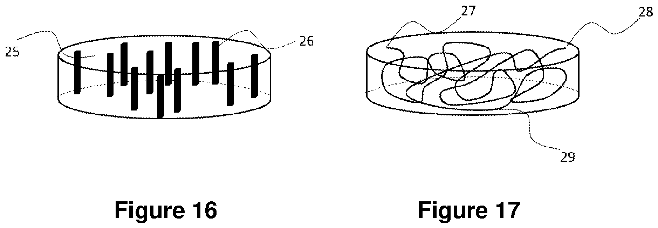

Figure 16 shows an embodiment including columnar cavities (26) for nanoparticle samples that can be filled and emptied quickly.

Figure 17 shows an embodiment which includes a thin tube (29) in the form of a complex curve in three dimensions with single input for filling (27) and an output for draining (28) the magnetic nanoparticles inside the calibration scene. The calibration scene may include a single or plural number of tubes of arbitrary path traversing the calibration scene for filling or emptying the tubes with nanoparticles.

## References

[1] Weizenecker J, Gleich B, Rahmer J, Dahnke H, Borgert J (2009) . Three-dimensional real-time in vivo magnetic particle imaging, Phys Med Biol. 2009;54: L1-L10.

[2] A. von Gladiss, M. Graeser, P. Szwargulski, T. Knopp and T. M. Buzug. Hybrid system calibration for multidimensional magnetic particle imaging. Phys. Med. Biol., vol. 62, no. 9, pp. 3392, 2017.

[3] Compressed Sensing Theory and Applications, Ed. By Y. C. Eldar, G. Kutyniok, Cambridge University Press, New York, 2012.

[4] B. Gleich and J. Weizenecker. Tomographic imaging using the nonlinear response of magnetic particles. Nature, 435(7046):1217-1217, 2005. doi: 10.1038/nature03808.

[5] T. Knopp, J. Rahmer, T. F. Sattel, S. Biederer, J. Weizenecker, B. Gleich, J. Borgert, and T. M. Buzug. Weighted iterative reconstruction for magnetic particle imaging. Phys. Med. Biol., vol. 55, no. 6, pp. 1577-1589, 2010. doi: 1 0.1 088/0031-9155/55/6/003.

[6] G. R. Arce, D. J. Brady, L. Carin, H. Arguello, and D. S. Kittle, "Compressive Coded Aperture Spectral Imaging," IEEE Signal Processing Magazine, vol. 31, no. 1,pp. 105-115, 2014.

[7] S. Ilbey et al., "Comparison of system-matrix-based and projection-based reconstructions for field free line magnetic particle imaging," International Journal on Magnetic Particle Imaging, vol. 3, no. 1, 2017.

[8] H. E Güven, A. Güngör, and M. Cetin, "An Augmented Lagrangian Method for Complex-Valued Compressed SAR Imaging," IEEE Trans. Comput. Imaging, 2(3):235-250, 2016.

[9] S. Ilbey et al., "Image reconstruction for Magnetic Particle imaging using an Augmented Lagrangian Method", IEEE 14th International Symposium on Biomedical imaging, pp. 64-47, 2017, doi: 10.1109/ISBI.2017.7950469.

[10] S. Ilbey et al.: "Coded scenes for fast system calibration in magnetic particle imaging",2018 IEEE 15TH INTERNATIONAL SYMPOSIUM ON BIOMEDICAL IMAGING (ISBI 2018), IEEE, 4 April 2018 (2018-04-04), pages 315-318, DOI: 10.1109/ISBI.2018.8363582

**Claims**

1. A calibration method for a magnetic particle imaging system to perform magnetic particle imaging of a field of view including the steps of;

   • moving a calibration scene:

      ◦ which has distributed nanoparticle samples inside its volume, larger than the field of view of the MPI system, and
      ◦ which includes a single tube of arbitrary path traversing the calibration scene, for filling or emptying the tube with nanoparticles,
      ◦ and wherein the moving is linearly in one or more direction(s) and/or rotating about one or more axes of the calibration scene by means of a mechanical system,

   • scanning the field free region in the field of view and acquiring calibration measurement data at a plurality of positions of the calibration scene,
   • reconstructing the system matrix with compressed sensing methods by using the measurement data and the position information of the calibration scene during data acquisition.

2. The calibration method for magnetic particle imaging system according to claim 1, including the step of reconstructing the system matrix using the following optimization problem subject to an inequality:

$$\underset{A}{\operatorname{argmin}} \|DA^T\|_1, subject\ to\ \|PA^T - A_p\|_2 < \varepsilon_p$$

   where $P$ is the nanoparticle density distribution in the field of view at each measurement position; $D$ is the matrix associated with a sparsifying transform for $A$, the system matrix; $A_p$ is the measurement matrix converted to Fourier space for each measurement position of the coded calibration scene; $\varepsilon_p$ represents a constant related to the error caused by the system noise.

3. The calibration method of claim 1, wherein the calibration scene is moved or rotated continuously.

4. The calibration method of claim 1, wherein the calibration scene includes a plurality of nanoparticle samples.

5. The calibration method of claim 1, wherein the nanoparticles in the calibration scene are distributed randomly or pseudo-randomly.

6. The calibration method of claim 1, wherein the nanoparticles in the calibration scene are connectively distributed for filling and emptying from two ends.

7. The calibration method of claim 1, wherein the position of the calibration scene is continuously monitored using a tracking device to measure the position of the calibration scene during data acquisition.

8. A calibration apparatus for magnetic particle imaging system, including;

   • a calibration scene with distributed nanoparticle samples inside its volume, larger than the field of view of the MPI system,
   • a mechanical system performing linear movements in one or more directions and/or rotational movements around one or more axes of the calibration scene,

   and wherein the calibration scene includes a single tube of arbitrary path traversing the calibration scene, for filling or emptying the tube with nanoparticles.

9. The calibration apparatus of claim 8, wherein the outer geometry of the calibration scene is a rectangular prism, cylinder, sphere or any arbitrary shape.

10. The calibration apparatus of claim 8, wherein one or more reflectors are attached to the calibration scene for tracking its movement.

11. The calibration apparatus of claim 8, wherein the calibration scene includes plural number of tubes traversing the calibration scene, for filling or emptying the tubes with nanoparticles.

12. The calibration apparatus of claim 8, wherein the calibration scene is a hollow structure with one or more openings for filling or emptying the structure with nanoparticles.

13. The calibration apparatus of claim 8, wherein its position is tracked by means of a tracking device.

14. The calibration apparatus of claim 8, wherein the mechanical system includes a control unit that communicates with the MPI system to carry out the operations for the calibration method.

**Patentansprüche**

1. Kalibrierungsverfahren für ein Magnetpartikelbildgebungssystem zum Ausführen von Magnetpartikelbildgebung eines Sichtfelds, das die folgenden Schritte beinhaltet,

   • Bewegen einer Kalibrierungsszene:

   o die verteilte Nanopartikelproben in ihrem Volumen aufweist, das größer ist als das Sichtfeld des MPI-Systems, und
   o die eine einzelne Röhre mit beliebigem Verlauf beinhaltet, die die Kalibrierungsszene durchquert, um die Röhre mit Nanopartikeln zu füllen oder zu entleeren,
   o und wobei das Bewegen linear in eine oder mehrere Richtungen und/oder ein Drehen um eine oder mehrere Achsen der Kalibrierungsszene mittels eines mechanischen Systems ist,

   • Scannen des feldfreien Bereichs in dem Sichtfeld und Erfassen von Kalibrierungsmessdaten an einer Vielzahl von Positionen der Kalibrierungsszene,
   • Rekonstruieren der Systemmatrix mit komprimierten Abtastverfahren unter Verwendung der Messdaten und der Positionsinformationen der Kalibrierungsszene während einer Datenerfassung.

2. Kalibrierungsverfahren für ein Magnetpartikelbildgebungssystem nach Anspruch 1, das den Schritt eines Rekonstruierens der Systemmatrix unter Verwendung des folgenden Optimierungsproblems beinhaltet, das von einer Ungleichheit abhängt:

$$\underset{A}{\operatorname{argmin}} \|DA^T\|_1, \quad \text{abhängig von} \quad \|PA^T - A_p\|_2 < \varepsilon_p$$

wobei $P$ die Nanopartikeldichteverteilung in dem Sichtfeld an jeder Messposition ist; $D$ die Matrix ist, die mit einer sparsifizierenden Transformation für $A$, die Systemmatrix, assoziiert ist; $A_p$ die in den Fourier-Raum konvertierte Messmatrix für jede Messposition der kodierten Kalibrierungsszene ist; $\varepsilon_p$ eine Konstante darstellt, die sich auf den durch das Systemrauschen verursachten Fehler bezieht.

3. Kalibrierungsverfahren nach Anspruch 1, wobei die Kalibrierungsszene kontinuierlich bewegt oder gedreht wird.

4. Kalibrierungsverfahren nach Anspruch 1, wobei die Kalibrierungsszene eine Vielzahl von Nanopartikelproben beinhaltet.

5. Kalibrierungsverfahren nach Anspruch 1, wobei die Nanopartikel in der Kalibrierungsszene zufällig oder pseudozufällig verteilt sind.

**6.** Kalibrierungsverfahren nach Anspruch 1, wobei die Nanopartikel in der Kalibrierungsszene zum Füllen und Leeren von zwei Enden aus verbindend verteilt sind.

**7.** Kalibrierungsverfahren nach Anspruch 1, wobei die Position der Kalibrierungsszene unter Verwendung einer Verfolgungseinrichtung zum Messen der Position der Kalibrierungsszene während der Datenerfassung kontinuierlich überwacht wird.

**8.** Kalibrierungsvorrichtung für Magnetpartikelbildgebungssystem, das Folgendes beinhaltet,

· eine Kalibrierungsszene mit verteilten Nanopartikelproben in ihrem Volumen, das größer ist als das Sichtfeld des MPI-Systems,
· ein mechanisches System, das lineare Bewegungen in einer oder mehreren Richtungen und/oder Drehbewegungen um eine oder mehrere Achsen der Kalibrierungsszene ausführt, und wobei die Kalibrierungsszene eine einzelne Röhre mit beliebigem Verlauf beinhaltet, das die Kalibrierungsszene durchquert, um das Rohr mit Nanopartikeln zu füllen oder zu leeren.

**9.** Kalibrierungsvorrichtung nach Anspruch 8, wobei die äußere Geometrie der Kalibrierungsszene ein rechteckiges Prisma, ein Zylinder, eine Kugel oder eine beliebige Form ist.

**10.** Kalibrierungsvorrichtung nach Anspruch 8, wobei ein oder mehrere Reflektoren an der Kalibrierungsszene angebracht sind, um deren Bewegung zu verfolgen.

**11.** Kalibrierungsvorrichtung nach Anspruch 8, wobei die Kalibrierungsszene eine Vielzahl von Röhren beinhaltet, die die Kalibrierungsszene durchqueren, um die Röhren mit Nanopartikeln zu füllen oder zu leeren.

**12.** Kalibrierungsvorrichtung nach Anspruch 8, wobei die Kalibrierungsszene eine hohle Struktur mit einer oder mehreren Öffnungen ist, um die Struktur mit Nanopartikeln zu füllen oder zu leeren.

**13.** Kalibrierungsvorrichtung nach Anspruch 8, wobei ihre Position mittels einer Verfolgungseinrichtung verfolgt wird.

**14.** Kalibrierungsvorrichtung nach Anspruch 8, wobei das mechanische System eine Steuereinheit beinhaltet, die mit dem MPI-System kommuniziert, um die Vorgänge für das Kalibrierungsverfahren durchzuführen.

**Revendications**

**1.** Procédé d'étalonnage pour un système d'imagerie à particules magnétiques pour réaliser une imagerie à particules magnétiques d'un champ de vision comprenant les étapes de ;

· déplacement d'une scène d'étalonnage :

◦ qui a distribué des échantillons de nanoparticules dans son volume, plus grand que le champ de vision du système MPI, et
◦ qui comprend un tube unique de trajet arbitraire traversant la scène d'étalonnage, pour remplissage ou vidage du tube avec des nanoparticules,
◦ et dans lequel le déplacement est linéaire dans une ou plusieurs directions et/ou tourne autour d'un ou plusieurs axes de la scène d'étalonnage au moyen d'un système mécanique,

· balayage de la région sans champ dans le champ de vision et acquisition de données de mesure d'étalonnage à une pluralité de positions de la scène d'étalonnage,
· reconstruction de la matrice du système avec des procédés de détection compressée à l'aide des données de mesure et des informations de position de la scène d'étalonnage pendant l'acquisition de données.

**2.** Procédé d'étalonnage pour système d'imagerie à particules magnétiques selon la revendication 1, comprenant l'étape de reconstruction de la matrice du système en utilisant le problème d'optimisation suivant soumis à une inégalité :

$$\underset{A}{\operatorname{argmin}} \|DA^T\|_1 \text{, soumis à } \|PA^T - A_p\|_2 < \varepsilon_p$$

où P est la distribution de la densité des nanoparticules dans le champ de vision à chaque position de mesure ; D est la matrice associée à une transformée sparsifiante pour A, la matrice du système ; $A_p$ est la matrice de mesure convertie en espace de Fourier pour chaque position de mesure de la scène d'étalonnage codée ; $\varepsilon_p$ représente une constante liée à l'erreur provoquée par le bruit du système.

3. Procédé d'étalonnage selon la revendication 1, dans lequel la scène d'étalonnage est déplacée ou tournée en continu.

4. Procédé d'étalonnage selon la revendication 1, dans lequel la scène d'étalonnage comprend une pluralité d'échantillons de nanoparticules.

5. Procédé d'étalonnage selon la revendication 1, dans lequel les nanoparticules dans la scène d'étalonnage sont distribuées de manière aléatoire ou pseudo-aléatoire.

6. Procédé d'étalonnage selon la revendication 1, dans lequel les nanoparticules dans la scène d'étalonnage sont distribuées de manière connective pour remplissage et vidage depuis deux extrémités.

7. Procédé d'étalonnage selon la revendication 1, dans lequel la position de la scène d'étalonnage est surveillée en continu à l'aide d'un dispositif de suivi pour mesurer la position de la scène d'étalonnage pendant l'acquisition de données.

8. Appareil d'étalonnage pour système d'imagerie à particules magnétiques, comprenant ;

   • une scène d'étalonnage avec des échantillons de nanoparticules distribués à l'intérieur de son volume, plus grande que le champ de vision du système MPI,
   • un système mécanique effectuant des mouvements linéaires dans une ou plusieurs directions et/ou des mouvements de rotation autour d'un ou plusieurs axes de la scène d'étalonnage, et dans lequel la scène d'étalonnage comprend un tube unique de trajet arbitraire traversant la scène d'étalonnage, pour remplissage ou vidage du tube avec des nanoparticules.

9. Appareil d'étalonnage selon la revendication 8, dans lequel la géométrie extérieure de la scène d'étalonnage est un prisme rectangulaire, un cylindre, une sphère ou toute forme arbitraire.

10. Appareil d'étalonnage selon la revendication 8, dans lequel un ou plusieurs réflecteurs sont fixés à la scène d'étalonnage pour suivre son déplacement.

11. Appareil d'étalonnage selon la revendication 8, dans lequel la scène d'étalonnage comprend plusieurs tubes traversant la scène d'étalonnage, pour remplissage ou vidage des tubes avec des nanoparticules.

12. Appareil d'étalonnage selon la revendication 8, dans lequel la scène d'étalonnage est une structure creuse avec une ou plusieurs ouvertures pour remplissage ou vidage de la structure avec des nanoparticules.

13. Appareil d'étalonnage selon la revendication 8, dans lequel sa position est suivie au moyen d'un dispositif de suivi.

14. Appareil d'étalonnage selon la revendication 8, dans lequel le système mécanique comprend une unité de commande qui communique avec le système MPI pour effectuer les opérations pour le procédé d'étalonnage.

**Figure 1**

**Figure 2**

**Figure 3**

Reference (N=80x40=3200)

Standard Compressed Detection

M=2560 M=1600 M=640

Proposed Method

M=320 M=160 M=97

**Figure 4**

**Figure 5**

**Figure 6**

**Figure 7**

**Figure 8**

**Figure 9**

**Figure 10**

**Figure 11**

**Figure 12**

**Figure 13**

**Figure 14**

**Figure 15**

Figure 16

Figure 17

## REFERENCES CITED IN THE DESCRIPTION

**Patent documents cited in the description**

- US 8355771 B2
- US 20150221103 A1
- EP 3143929 A1
- US 20170020407 A1

**Non-patent literature cited in the description**

- **WEIZENECKER J ; GLEICH B ; RAHMER J ; DAHNKE H ; BORGERT J.** Three-dimensional real-time in vivo magnetic particle imaging. *Phys Med Biol,* 2009, vol. 54, L1-L10
- **A. VON GLADISS ; M. GRAESER ; P. SZWARGULSKI ; T. KNOPP ; T. M. BUZUG.** Hybrid system calibration for multidimensional magnetic particle imaging. *Phys. Med. Biol.,* 2017, vol. 62 (9), 3392
- Compressed Sensing Theory and Applications. Cambridge University Press, 2012
- **B. GLEICH ; J. WEIZENECKER.** Tomographic imaging using the nonlinear response of magnetic particles. *Nature,* 2005, vol. 435 (7046), 1217-1217
- **T. KNOPP ; J. RAHMER ; T. F. SATTEL ; S. BIEDERER ; J. WEIZENECKER ; B. GLEICH ; J. BORGERT ; T. M. BUZUG.** Weighted iterative reconstruction for magnetic particle imaging. *Phys. Med. Biol,* 2010, vol. 55 (6), 1577-1589
- **G. R. ARCE ; D. J. BRADY ; L. CARIN ; H. ARGUELLO ; D. S. KITTLE.** Compressive Coded Aperture Spectral Imaging. *IEEE Signal Processing Magazine,* 2014, vol. 31 (1), 105-115
- **S. ILBEY et al.** Comparison of system-matrix-based and projection-based reconstructions for field free line magnetic particle imaging. *International Journal on Magnetic Particle Imaging,* 2017, vol. 3 (1
- **H. E GÜVEN ; A. GÜNGÖR ; M. CETIN.** An Augmented Lagrangian Method for Complex-Valued Compressed SAR Imaging. *IEEE Trans. Comput. Imaging,* 2016, vol. 2 (3), 235-250
- **S. ILBEY et al.** Image reconstruction for Magnetic Particle imaging using an Augmented Lagrangian Method. *IEEE 14th International Symposium on Biomedical imaging,* 2017, 64-47
- Coded scenes for fast system calibration in magnetic particle imaging. **S. ILBEY et al.** 2018 IEEE 15TH INTERNATIONAL SYMPOSIUM ON BIOMEDICAL IMAGING (ISBI 2018). IEEE, 04 April 2018, 315-318